(19) Europäisches Patentamt
European Patent Office
Office européen des brevets

(11) Veröffentlichungsnummer: **0 212 137 B1**

(12) # EUROPÄISCHE PATENTSCHRIFT

(45) Veröffentlichungstag der Patentschrift: **26.08.92**

(51) Int. Cl.[5]: **A61K 31/015**

(21) Anmeldenummer: **86108548.8**

(22) Anmeldetag: **23.06.86**

(54) **Verwendung eines Retinoids zur Herstellung eines zytostatischen Arzneimittels.**

(30) Priorität: **28.06.85 CH 2755/85**

(43) Veröffentlichungstag der Anmeldung:
**04.03.87 Patentblatt 87/10**

(45) Bekanntmachung des Hinweises auf die Patenterteilung:
**26.08.92 Patentblatt 92/35**

(84) Benannte Vertragsstaaten:
**BE CH DE FR GB IT LI LU NL SE**

(56) Entgegenhaltungen:

**EUR. J. MED. CHEM.-CHIMTER, Band 15, Nr. 1, 1980, Seiten 9-15; P. LOFLIGER et al.: "Arotinoids, a new class of highly active retinoids"**

**JOURNAL OF THE AMERICAN ACADEMY OF DERMATOLOGY, Band 9, Nr. 5, November 1983, Seiten 797-805; W. BOLLAG: "The development of retinoids in experimental and clinical oncology and dermatology"**

(73) Patentinhaber: **F. HOFFMANN-LA ROCHE AG**
**Postfach 3255**
**CH-4002 Basel(CH)**

(72) Erfinder: **Bollag, Werner, Dr.**
**Mythenstrasse 10**
**CH-4054 Basel(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

BRIT. J. HAEMATOL, Band 5911, 1985, Seiten 15-20; R. BAILEY-WOOD et al.: "The effect of retinoids on CFU-GM from normal subjects and patients with myelodysplastic syndrome"

DTSCH. MED. WSCHR., Band 108, Nr. 46, 1983, Seiten 1753-1757, Georg Thieme Verlag, Stuttgart, DE; G. MAHRLE et al.: "Chemotherapie kutaner T-Zell-Lymphome mit Arotinoid"

"Retinoids: New Trends in Research and Therapy", Saurel (ed.), Retinoid Symp., Geneva, 1984, pages 48-54, Karger, Basel, CH; H. HEMMI et al.: "Induction of NAD+-Glycohydrolase. A rapid assay for differentiation of a human promyelocytic cell line (HL-60) by retinoids"

CANCER RESEARCH, Band 43, September 1983, Seiten 4283-4290; M.I. SHERMAN et al.: "Effects of arotinoids upon murine embryonal carcinoma cells"

## Beschreibung

Im Rahmen der vorliegenden Erfindung wurde festgestellt, dass das 1,2,3,4-Tetrahydro-1,1,4,4-tetramethyl -6-(α-methylstyryl)naphthalin der Formel

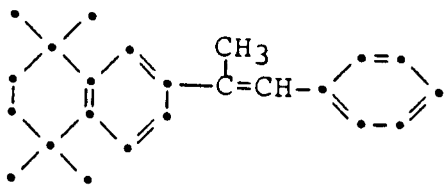

I

antihyperplastische, antimetaplastische, tumorpräventive und tumortherapeutische Eigenschaften besitzt. Es ist bekannt, die Verbindung der Formel I zur systemischen und topischen Behandlung bzw. Verhütung von Erscheinungen zu verwenden, die auf einer erhöhten Talg-Ausscheidung beruhen, wie fettiges Haar, fettige Kopfhaut, Seborrhoe und insbesondere Akne vulgaris.

In Eur. J. Med. Chem.-Chim. Ther., 15 (1) 1980, Seiten 9-15, wird mitgeteilt, dass sich die oben erwähnte Verbindung bei der Prüfung auf Hemmwirkung bei chemisch erzeugten Papillomen der Maus als inaktiv erwies.

Demzufolge überraschend wurde nun gefunden, dass durch systemische und topische Verabreichung der Verbindung der Formel I andere pathologische Zustände als die zuletzt erwähnten erfolgreich behandelt bzw. verhütet werden können. Die vorliegende Erfindung basiert auf dem eben genannten Befund und betrifft demnach die neue Verwendung der Verbindung der Formel I bei der Herstellung Arzneimitteln zur Behandlung bzw. Verhütung von Praekanzerosen, benignen und malignen Tumoren, epithelialer und mesenchymaler Natur. Beispiele solcher Praekanzerosen und benigner Tumoren sind aktinische Keratosen, Arsenkeratosen, Xeroderma pigmentosum, Morbus Bowen, Hyperkeratosen, Pachydermien, Leukoplakien, Metaplasien, Dysplasien und Papillome von Schleimhäuten, z.B. von Mund, Zunge, Pharynx und Larynx, praekanzeröse Veränderungen der Bronchialschleimhaut, wie Metaplasien und Dysplasien (besonders häufig bei schweren Rauchern und Leuten, die mit Asbest- und/oder Uranium arbeiten), Dysplasien und Leukoplakien von Cervix uteri und Vulva, Kraurosis vulvae, praekanzeröse Veränderungen der Harnblase, z.B. Metaplasien, Dysplasien und Papillome, sowie Polypen des Magendarmtraktes. Beispiele von Tumoren, semimaligner oder maligner Natur, epithelialer oder mesenchymaler Herkunft sind Mammatumoren, Hauttumoren, z.B. Basalzellkarzinome, Blasentumoren, z.B. oberflächliche Blasenkarzinome, Dickdarmtumoren, Speiseröhrentumoren, Magentumoren, Larynxtumoren, Lungentumoren, B- und T-Zellymphome, Chondrosarkome und Osteosarkome.

Die Behandlung bzw. Verhütung benigner und maligner Tumoren epithelialer und mesenchymaler Natur kann mit der Verbindung der Formel I allein oder in Kombination mit anderen Massnahmen, wie Chirurgie, Strahlenbehandlung, Hormonbehandlung oder Behandlung mit Zytostatika, Interferonen, Lymphokinen etc., erfolgen.

Für die erfindungsgemässe Verwendung ist das (E)-Isomere, d.h. das 1,2,3,4-Tetrahydro-1,1,4,4-tetramethyl -6-[(E)-α-methylstyryl]naphthalin (Verbindung Ia), bevorzugt, obwohl vorauszusehen ist, dass das (Z)-Isomere, d.h. das 1,2,3,4-Tetrahydro-1,1,4,4-tetramethyl -6-[(Z)-α-methylstyryl]naphthalin (Verbindung Ib) im Rahmen der vorliegenden Erfindung ebenfalls verwendet werden kann, sei es allein oder in Kombination mit dem bevorzugten (E)-Isomeren Ia.

Zur Behandlung bzw. Verhütung der oben genannten Krankheiten wird die Verbindung der Formel I systemisch oder topisch, vorzugsweise systemisch, besonders bevorzugt enteral, ganz besonders bevorzugt oral, verabreicht.

Die Dosis bei der systemischen Verabreichung variiert gemäss den Bedürfnissen des einzelnen Patienten, wie sie vom behandelnden Arzt bestimmt worden sind. Im allgemeinen dürfte jedoch eine tägliche Dosis von etwa 1 mg bis etwa 50 mg, vorzugsweise von etwa 3 mg bis etwa 15 mg, pro kg Körpergewicht des Patienten zum Einsatz gelangen. Die Dosis kann als Einzeldosis oder auf mehrere Teildosen verteilt gemäss einem Dosierungsplan, wie er vom Arzt gemäss den Bedürnissen des Patienten bestimmt wird, verabreicht werden.

Als Verabreichungsform kommen die für die systemische Verabreichung üblichen festen oder flüssigen Darreichungsformen in Betracht, z.B. Suppositorien oder als feste orale Darreichungsformen Kapseln, Tabletten, Dragées, Pillen, Puder, Granulate und dergleichen, als flüssige orale Darreichungsformen Lösungen, Sirupe, Suspensionen, Elixire und dergleichen und als parenterale Darreichungsformen Infusions-

oder Injektionslösungen, die intravenös oder intramuskulär injiziert werden können.

Es liegt im Bereich der vorliegenden Erfindung, die Verbindung der Formel I in jeder für die systemische Verabreichung geeigneten Menge in die enterale oder parenterale Darreichungsform einzuarbeiten. Es ist jedoch bevorzugt, Präparate herzustellen, die den erfindungsgemässen Wirkstoff in einer Menge von etwa 50-1000 mg, vorzugsweise von etwa 150-500 mg, enthalten. Besonders bevorzugt ist die Herstellung von Kapseln und Tabletten.

Zur topischen Verabreichung geeignet sind Lösungen, Lotions, Suspensionen, Salben, Crèmes, Gels, mikronisierte Puder, Aerosole und dergleichen. Diese Präparate enthalten zweckmässig 0,1-10 Gew.%, vorzugsweise 0,5-5 Gew.% der Verbindung der Formel I berechnet auf das Gesamtgewicht des Präparates.

Die Herstellung der oben genannten systemischen und topischen Gebrauchsformen kann in üblicher Weise, z.B. anhand der nachstehenden Beispiele erfolgen.

Beispiel 1

Hartgelatinekapseln enthaltend die folgenden Bestandteile können hergestellt werden:

| Bestandteile | mg/Kapsel |
|---|---|
| 1. sprühgetrocknetes Pulver enthaltend 75% Verbindung Ia | 200 |
| 2. Natriumdioctylsulfosuccinat | 0,2 |
| 3. Natriumcarboxymethylcellulose | 4,8 |
| 4. mikrokristalline Cellulose | 86.0 |
| 5. Talk | 8,0 |
| 6. Magnesiumstearat | 1,0 |
| Total | 300 |

Verfahren:

Das sprühgetrocknete Pulver, das auf dem Wirkstoff, Gelatine und mikrokristalliner Cellulose basiert und eine mittlere Korngrösse des Wirkstoffes von <1 μ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel 2

Tabletten, enthaltend die folgenden Bestandteile, können hergestellt werden:

| Bestandteile | mg/Tablette |
|---|---|
| 1. Verbindung Ia als feingemahlenes Pulver | 500 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| Total | 800 |

Verfahren:

Die feingemahlene Substanz wird mit Milchzucker pulv. und Maisstärke weiss gemischt. Die Mischung wird mit einer wässrigen Lösung von Povidone K30 befeuchtet und geknetet, und die resultierende Masse granuliert, getrocknet und gesiebt. Das Granulat wird mit Maisstärke weiss (2. Teil), Talk und Magnesiumstearat vermischt und zu Tabletten geeigneter Grösse verpresst.

Beispiel 3

Weichgelatinekapseln, enthaltend die folgenden Bestandteile, können hergestellt werden:

| Bestandteile | mg/Kapsel |
|---|---|
| 1. Verbindung Ia | 50 |
| 2. Triglycerid | 450 |
| Total | 500 |

Verfahren:

10 g Verbindung Ia werden unter Rühren, Inertbegasung und Lichtschutz in 90 g mittelkettigem Triglycerid gelöst. Diese Lösung wird als Kapselfüllmasse von einem Vertragshersteller zu Weichgelatinekapseln à 50 mg Wirkstoff verarbeitet.

Beispiel 4

Eine Fettsalbe, enthaltend die folgenden Bestandteile, kann hergestellt werden:

| Bestandteile | |
|---|---|
| 1. Verbindung Ia, feingemahlen | 3,0 g |
| 2. Paraffinöl, intern dickflüssig | 30,0 g |
| 3. Lunacera M | 15,0 g |
| 4. Ricinusöl, gehärtet | 5,0 g |
| 5. Vaseline, weiss | ad 100 g |

Verfahren:

Sämtliche Hilfsstoffe werden in der Wärme gemischt und auf Raumtemperatur kaltgerührt. Der Wirkstoff wird kalt unter Lichtschutz mit der auf diese Weise erhaltenen Mischung homogen angerieben.

Beispiel 5

Eine Fettcrème, enthaltend die folgenden Bestandteile, kann hergestellt werden:

| Bestandteile | | |
|---|---|---|
| 1. Verbindung Ia, feingemahlen | 3,0 g | |
| 2. Vaseline, weiss | 30,0 g | Fettphase |
| 3. Wachs, weiss | 5,0 g | Fettphase |
| 4. Paraffinöl, intern dickflüssig | 20,0 g | Fettphase |
| 5. Dehymuls E | 9,0 g | Wasserphase |
| 6. Benzoesäure | 0,2 g | Wasserphase |
| 7. entmineralisiertes Wasser | ad 100,0 g | Wasserphase |

Verfahren:

Fett- und Wasserphase werden zu einer Fettcrème verarbeitet. Der Wirkstoff wird bei Raumtemperatur unter Lichtschutz mit dieser Fettcrème homogen angerieben.

Beispiel 6

Eine Vanishingcrème (Typ O/W-Emulsion), enthaltend die folgenden Bestandteile, kann hergestellt werden:

| Bestandteile | | |
|---|---|---|
| 1. Verbindung Ia, feingemahlen | 3,0 g | |
| 2. Glycerinmonostearat | 17,0 g | Fettphase |
| 3. Deltyl extra | 4,0 g | Fettphase |
| 4. Tween 60 | 4,0 g | Fettphase |
| 5. Span 60 | 4,0 g | Fettphase |
| 6. Silikonöl AR 20 | 2,0 g | Fettphase |
| 7. Propylenglykol | 10,0 g | Wasserphase |
| 8. Benzoesäure, rein | 0,2 g | Wasserphase |
| 9. entmineralisiertes Wasser | ad 100,0 g | Wasserphase |

Verfahren:

Fett- und Wasserphase werden zu einer Crème verarbeitet. Der Wirkstoff wird bei Raumtemperatur unter Lichtschutz mit dieser Crème homogen angerieben.

Beispiel 7

Ein hydrophiles Gel, enthaltend die folgenden Bestandteile, kann hergestellt werden:

| Bestandteile | |
|---|---|
| 1. Verbindung Ia, feingemahlen | 3,0 g |
| 2. Carbopol 940 | 2,5 g |
| 3. Propylenglykol | 50,0 g |
| 4. Aethanol, 94% | ad 100,0 g |

Verfahren:

Der Wirkstoff wird unter Lichtschutz in die Mischung Propylenglykol/Aethanol, 94%ig, eingearbeitet. Carbopol 940 wird bis zu vollständigen Gelierung eingerührt.

Beispiel 8

Eine Lotion, enthaltend die folgenden Bestandteile, kann hergestellt werden:

| Bestandteile | |
|---|---|
| 1. Verbindung Ia, feingemahlen | 3,0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Verfahren:

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

Die therapeutische und prophylaktische Wirkung der Verbindung der Formel I bei Praekanzerosen und Tumoren epithelialer und mesenchymaler Natur kann den nachfolgenden Versuchen entnommen werden:

A) Die Wirkung der Verbindung Ia bezüglich Verhütung chemisch induzierter Mammatumoren wurde nach dem folgenden Prozedere bestimmt. Weibliche Sprague-Dawley-Ratten (Madörin AG, Füllinsdorf/Schweiz) wurden in diesem Versuch eingesetzt. Die Versuchstiere wurden unter Temperatur- und Licht-kontrollierten Bedingungen gehalten und hatten freien Zugang zu Trinkwasser und Futter. Im Alter von 50 Tagen wurde jeder Ratte 15 mg von in Arachisöl aufgelöstem Dimethylbenz(a)anthracen (Fluka AG, Buchs/Schweiz) mittels einer Magensonde verabreicht. Die Behandlung mit der Verbindung Ia begann 1 Tag nach der Verabreichung des Karzinogens. Das Körpergewicht der Versuchstiere wurde aufgezeichnet und die Tumoren wöchentlich palpiert und mit einer Schublehre ausgemessen. Die Volumina wurden nach der Formel $\frac{D}{2} \cdot d^2$ berechnet, wobei D der grössere und d der kleinere Durchmesser des Tumorellipsoids darstellt. Der Versuch wurde nach 11 Wochen beendet und ausgewertet. In diesem Versuch wurden neben den 30 Kontrolltieren, welche ausschliesslich normales Futter erhielten die folgenden drei Gruppen von Versuchstieren eingesetzt:

1. 33 Ratten, denen mit dem Futter vermischt täglich 30 mg/kg Verbindung Ia verabreicht wurden.
2. 36 Ratten, denen mit dem Futter vermischt täglich 90 mg/kg Verbindung Ia verabreicht wurden.
3. 33 Ratten, denen mit dem Futter vermischt täglich 270 mg/kg Verbindung Ia verabreicht wurden.

Das Futtergemisch wurde wöchentlich nach dem Körpergewicht und der totalen Nahrungsaufnahme justiert. Die Resultate sind in der Tabelle I zusammengefasst.

Tabelle I

| tägliche orale Dosis | % tumortragende Ratten | Durchschnittliche Anzahl von Tumoren pro Ratte | Durchschnittliches Tumorvolumen pro Ratte in $mm^3$ |
|---|---|---|---|
| Kontrolltiere | 79,3 (100%) | 3,7 (100%) | 6335 (100%) |
| 30 mg/kg | 69,6 ( 88%) | 3,0 ( 81%) | 4904 ( 77%) |
| 90 mg/kg | 63,8 ( 80%) | 1,8 ( 48%) | 2879 ( 45%) |
| 270 mg/kg | 39,3 ( 50%) | 0,7 ( 18%) | 543 ( 9%) |

B) Die Wirkung der Verbindung Ia auf Tumoren wurde weiterhin am transplantablen Chondrosarkom der Ratte nach der folgenden Methode bestimmt. Der feste Tumor eines Spendertiers wurde fein gehackt und in Phosphat gepufferter Kochsalzlösung suspendiert. 0,5 ml des 30%igen Tumorbreis wurde Albinoratten (Füllinsdorf) subkutan implantiert. Der Versuch wurde gestartet, wenn die Versuchstiere eine Tumorfläche (Produkt aus grösstem und kleinstem Durchmesser) von ungefähr 1200-1300 $mm^2$ aufwie-

sen (3-4 Wochen nach der Implantation). Jede Behandlungsgruppe umfasste 7 Ratten. Suspensionen von Verbindung Ia in Arachisöl wurden während 4 Wochen fünfmal wöchentlich oral verabreicht. Die Tumorflächen wurden an den Tagen 1, 15 und 29 bestimmt. Der grösste (D) und der kleinste (d) Durchmesser der Tumoren wurden mit Hilfe einer Schublehre bestimmt und das Produkt D•d berechnet. Die Aenderung dieser Flächengrösse wurde in Prozenten berechnet. Die Resultate sind in der Tabelle II zusammengefasst.

Tabelle II

| Behandlung | Tumorfläche in $mm^2$ Tag 1 | Tag 15 | Tag 29 |
|---|---|---|---|
| | nach Behandlungsbeginn | | |
| Kontrolltiere | 1199 | 2880 (+140%) | 5926 (+394%) |
| Verbindung Ia 400 mg/kg p.o. | 1300 | 1416 (+ 9%) | 1694 (+ 30%) |

C) Die antimetaplastische Wirkung der Verbindung Ia wurde nach der folgenden Methode bei Ratten bestimmt. Weibliche Holtzmann-Ratten (Füllinsdorf/Schweiz) mit einem Gewicht von ca. 100 g wurden nach einer Eingewöhnungszeit von 8 Tagen unter Thiogenalnarkose ovarektomiert und nach weiteren 14 Tagen in den Versuch genommen. Je zwei Tiere wurden in einem Käfig untergebracht, und hatten freien Zugang zum Futter, das ca. 2000 IE analytisch bestimmtes Vitamin A enthielt. Vor der oralen Verabreichung der Testverbindung wurden die Tiere an 6 aufeinanderfolgenden Tagen täglich mit dem Hormongemisch von 1 µg Oestradiolbenzoat und 250 µg Testosteronpropionat, gelöst in 0,1 ml Sesamöl subkutan behandelt. Die parenterale Hormonapplikation führt zur Ausbildung eines reinen Schollenstadiums im Vaginalabstrich, d.h. einer squamösen Metaplasie. 2 Tage nach der oralen Verabreichung der Testsubstanz wurde das Reaktionsergebnis wiederum am Vaginalepithel abgelesen. Für die Berechnung der mittleren wirksamen Dosen wurde die Flächenmethode nach Behrens und Kärber herangezogen. Die Resultate sind in der Tabelle III zusammengefasst.

Tabelle III

| Verbindung | Antimetaplastischer Effekt | Relative Aktivität |
|---|---|---|
| all-trans-Vitamin A-säure | 35,7 µg | 100 % |
| Verbindung Ia | 53,6 µg | 66,6% |

Wie den obigen Resultaten entnommen werden kann, wurde für das Standardpräparat eine mittlere Schutzdosis von 35,7 µg ermittelt, während die Schutzdosis für die Testverbindung 53,6 µg betrug. Für die Verbindung Ia wurde demnach ein um den Faktor 0,67 niedrigerer Epithelschutzeffekt (= antimetaplastischer Effekt) gefunden als beim gewählten Standardpräparat all-trans Vitamin A-säure.

Da Metaplasie als erster Schritt in der Transformation von normalem Gewebe zu prämalignem und malignem Gewebe betracht werden kann, lassen sich aufgrund der Metaplasie verhütenden Wirkung einer bestimmten Verbindung gewisse Voraussagen bezüglich der tumorverhütenden Wirkung dieser Verbindung machen.

D) Die Wirkung der Verbindung Ia auf chemisch-induzierte praekanzeröse Gewebeveränderungen wurde weiterhin nach der folgenden Methode in vitro bestimmt. In Organkulturen gezüchtete Prostatadrüsen von Mäusen und Luftröhren von Ratten dienten in diesem Versuch als experimentelle Modelle. Karzinogene Substanzen bewirkt in Geweben von Organkulturen nach kurzer Zeit praekanzeröse Veränderungen in Form von Hyperplasien, aber auch von Metaplasien und Dysplasien. Retinoide, welche gleichzeitig mit

den karzinogenen Verbindungen oder nach diesen verabreicht werden, verhindern die Hyperplasie bzw. Metaplasie und Dysplasie oder machen diese gar rückgängig.

Die Prostatadrüsen bestehen aus Alveolen besetzt mit einer Reihe sekretorischer Zellen und getrennt durch ein dünnes Bindegewebe. Sie wurden 3-5 Monate alten Mäusen entnommen und in Läppchen von ca. 1,5 x 1,5 x 2,0 mm geschnitten. Mindestens sechs solcher Läppchen wurden auf Filterpapier gelegt und auf Metallgitter in kleinen Kulturkammern verbracht. Zwei solcher Kulturkammern wurden in einer mit feuchtem Filterpapier ausgelegten Petrischale eingeschlossen. Die Kulturkammern wurden mit dem Nährmedium bis zur Höhe der Gitter gefüllt, sodass die Explantate mit dem Nährmedium gut befeuchtet waren, jedoch nicht in dieses eintauchten. Als Nährmedium wurde gewöhnlich Medium 199 verwendet, ergänzt durch Zugabe von 15% Serum von frisch geborenen Kälbern. Das Karzinogen (3-Methylcholanthren) wurde in einer Konzentration von 4-5 µg/ml Nährmedium diesem vor der Inkubation zugegeben. Die Kulturen wurden dann bei 37,5 °C inkubiert. Während der Inkubation wurden sie in einer Macintosh-Flasche behalten, welche mit einem Gemisch von 5% $CO_2$ + 95% Sauerstoff begast wurde. In einer ersten Serie von Experimenten wurde das Karzinogen und die Verbindung Ia kombiniert und das Gewebe während 10-12 Tagen gezüchtet. In einer zweiten Serie wurden die Explantate mit dem Karzinogen während 10-12 Tagen vorbehandelt und nachher während 4-7 Tagen in ein Nährmedium verbracht, welches nur die Verbindung Ia enthielt. Die Konzentration an Verbindung Ia lag im Bereich von $10^{-9}$ bis $10^{-6}$ M.

Die Wirkung der Verbindung Ia wird durch Zählen der normalen und hyperplastischen Alveolen in Schnitten des Explantats quantifiziert. Die Resultate werden in Prozenten hyperplastischer Alveolen der Gesamtanzahl Alveolen ausgedrückt, beispielsweise 120 hyperplastische Alveolen von total 200 entsprechen 60%. In mit Retinoiden behandelten Explantaten ist der prozentuelle Anteil gewöhnlich kleiner. Beispielsweise zeigt ein mit einem Karzinogen behandeltes Explantat 60% und ein mit einem Retinoid behandeltes Explantat 20%. Der prozentuelle Anteil von hyperplastischen Alveolen ist auf 20/60 = 33% reduziert.

Luftröhren von Ratten werden in der gleichen Weise gezüchtet und mit Dimethylbenzanthracen als Karzinogen behandelt. Als Parameter der Hyperplasie dient der mitotische Index. Unter Retinoid wird der mitotische Index bzw. die Hyperplasie geringer. Die Resultate sind in den Tabellen IV (Prostata) und V (Luftröhre) zusammengefasst.

## Tabelle IV

Prostata (Maus)/durch Methylcholanthren induzierte Hyperplasie

| gleichzeitige Behandlung mit Verbindung Ia | | nachfolgende Behandlung mit Verbindung Ia |
|---|---|---|
| Methylcholanthren (47% hyperplastische Alveolen) | 100% | Methylcholanthren (65% hyperplastische Alveolen) 100% |
| Verbindung Ia $10^{-9}$M | 90% | 100% |
| $10^{-8}$M | 60% | 98% |
| $10^{-7}$M | 46% | 94% |
| $10^{-6}$M | 25% | 54% |

## Tabelle V

Luftröhren (Ratten)/durch Dimethylbenzanthracen induzierte Hyperplasie

| gleichzeitige Behandlung mit Verbindung Ia | | nachfolgende Behandlung mit Verbindung Ia |
|---|---|---|
| Dimethylbenzanthracen (Mitotischer Index 2,6) | 100% | Dimethylbenzanthracen (Mitotischer Index 3,1) 100% |
| Verbindung Ia $10^{-9}$M | 104% | 75% |
| $10^{-8}$M | 46% | 74% |
| $10^{-7}$M | 46% | 70% |
| $10^{-6}$M | 54% | 45% |

Es ist bekannt, dass das 1,2,3,4-Tetrahydro-1,1,4,4-tetramethyl -6-[(E)-α-methylstyryl]naphthalin Ia gut vertragen wird, und dass in den therapeutisch angezeigten Dosen keine toxischen Symptome auftreten. So zeigt die Verbindung Ia insbesondere keine Hypervitaminose A-Erscheinungen (z.B. keine Manifestationen an Haut und Schleimhäuten), und ist nicht teratogen und nicht hautirritierend. Darüber hinaus hat sich gezeigt, dass die Verbindung Ia, als einziges therapeutisch wirksames Retinoid, keine Erhöhung der Lipidwerte im Blutplasma verursacht, sondern vielmehr sogar eine lipidsenkende Wirkung besitzt.

Zum Nachweis dieser Wirkung wurden pro Dosis je 5 männlichen und weiblichen Albinoratten (Füllinsdorf/Schweiz) von ca. 150 g Gewicht mittels Schlundsonde innerhalb von 2 Tagen in Abständen von jeweils 18 und 6 Stunden fünfmal eine Dosis von Verbindung Ia verabreicht. 18 Stunden nach der letzten Applikation wurde den Versuchstieren retroorbital 1-1,2 ml Blutplasma entnommen. Der Triglyceridwert wurde durch enzymatische Spaltung der Triglyceride mit nachfolgender Bestimmung des entstandenen Glycerins (Farbreaktion) mittels Peridochrom Triglyceride GPO-PAP bestimmt. Die Resultate sind in der Tabelle VI zusammengefasst.

## Tabelle VI

| | männliche Ratten | weibliche Ratten |
|---|---|---|
| Kontrolltiere | 201±39 mg/100 ml | 69±28 mg/100 ml |
| Verbindung der Formel Ia | | |
| 10 mg/kg | 158±56 mg/100 ml | 56±25 mg/100 ml |
| 100 mg/kg | 173±18 mg/100 ml | 72±18 mg/100 ml |
| 1000 mg/kg | 41±19 mg/100 ml | 30± 8 mg/100 ml |

## Patentansprüche

1. Verwendung von 1,2,3,4-Tetrahydro-1,1,4,4-tetramethyl -6-(α-methylstyryl)naphthalin zur Herstellung eines Mittels zur Behandlung bzw. Verhütung von Praekanzerosen und benignen und malignen Tumoren epithelialer und mesenchymaler Natur.

2. Verwendung gemäss Anspruch 1 dadurch gekennzeichnet, dass es sich um Mamma-, Haut-, Blasen-, Dickdarm-, Speiseröhren-, Magen, Larynx- und Lungentumoren handelt.

3. Verwendung gemäss Anspruch 2, dadurch gekennzeichnet, dass es sich um einen Mammatumor handelt.

4. Verwendung gemäss einem der Ansprüche 1-3, dadurch gekennzeichnet, dass 1,2,3,4-Tetrahydro-1,1,4,4-tetramethyl -6-[(E)-α-methylstyryl]naphthalin verwendet wird.

## Claims

1. The use of 1,2,3,4-tetrahydro-1,1,4,4-tetramethyl-6-(α-methylstyryl)naphthalene for the manufacture of a medicament for the treatment or prevention of precanceroses and benign and malignant tumours of epithelial and mesenchymal nature.

2. The use in accordance with claim 1, wherein the tumour is a breast, skin, bladder, colon, esophagus, stomach, larynx or lung tumour.

3. The use in accordance with claim 2, wherein the tumour is a breast tumour.

4. The use in accordance with any one of claims 1-3, wherein 1,2,3,4-tetrahydro-1,1,4,4-tetramethyl-6-[(E)-α-methylstyryl]naphthalene is used.

## Revendications

1. Utilisation du 1,2,3,4-tétrahydro-1,1,4,4-tétraméthyl-6-(α -méthylstyryl)naphtalène pour préparer un agent pour le traitement ou la prévention des maladies précancéreuses et des tumeurs bénignes et malignes de nature épithéliale et mésenchymateuses.

2. Utilisation selon la revendication 1 , caractérisée en ce qu'il s'agit de tumeurs des glandes mammaires, de la peau, de la vessie, du gros intestin, de l'oesophage, de l'estomac, du larynx et des poumons.

3. Utilisation selon la revendication 2, caractérisée en ce qu'il s'agit d'une tumeur des glandes mammaires.

4. Utilisation selon l'une des revendications 1-3, caractérisée en ce qu'on emploie le 1,2,3,4-tétrahydro-1,1,4,4-tétraméthyl-6- [ (E)- α -méthylstyryl]naphtalène.